## Europäisches Patentamt
## European Patent Office
## Office européen des brevets

(11) Publication number: **0 219 282**
**A2**

(12) **EUROPEAN PATENT APPLICATION**

(21) Application number: 86307660.0

(22) Date of filing: 03.10.86

(51) Int. Cl.⁴: **C 07 C 45/64**
C 07 C 45/65, C 07 C 49/825
C 07 C 45/46, C 07 C 45/00
C 07 C 49/403

(30) Priority: 11.10.85 JP 227588/85

(43) Date of publication of application:
22.04.87 Bulletin 87/17

(84) Designated Contracting States:
DE GB

(71) Applicant: SUMITOMO CHEMICAL COMPANY, LIMITED
15 Kitahama 5-chome Higashi-ku
Osaka-shi Osaka 541(JP)

(72) Inventor: Tanaka, Kunihiko
5-41-201, Ayameikeminami-6-chome
Nara-shi(JP)

(74) Representative: Harrison, David Christopher et al,
MEWBURN ELLIS & CO 2/3 Cursitor Street
London EC4A 1BQ(GB)

(54) A method for producing 2-acetylresorcin.

(57) A method for producing 2-acetylresorcin useful as an important intermediate in the fields of, for example, medicines and agricultural chemicals comprises dehydrogenating 2-acetyl-1,3-cyclohexanedione by heating in a reaction medium in the presence of a palladium catalyst.

Croydon Printing Company Ltd

# A METHOD FOR PRODUCING 2-ACETYLRESORCIN

The present invention relates to a method for producing 2-acetylresorcin.

2-Acetylresorcin is an important intermediate in the fields of medicines, agricultural chemicals, etc., and for its production, there is known a method which uses resorcin as a starting material and passes through a 4-hydroxycoumarin route (Org. Synth. Coll., Vol. 3, 281).

This method, however, was not said to be an industrially advantageous method because there are many reaction steps and the yield is relatively low.

For this reason, the present inventors studied to produce 2-acetylresorcin in high yields and industrially advantageously, and as a result, found that 2-acetylresorcin can be obtained easily and in high yields by hydrogenating resorcin, a starting material, into 1,3-cyclohexanedione which is then acetylated into 2-acetyl-1,3-cyclohexanedione and dehydrogenating the resulting 2-acetyl-1,3-cyclohexanedione in a reaction medium in the presence of a palladium catalyst. The present inventors thus completed the present invention.

Every step of this method will be explained in detail.

1,3-Cyclohexanedione can be obtained in very high yields by hydrogenating resorcin in an aqueous alkali solution in the presence of a catalyst. In carrying out

this reaction, Raney nickel, ruthenium, rhodium, etc. are used as the catalyst, and ruthenium and rhodium are generally used as supported on various well-known carriers such as activated carbon, silica, alumina, etc. The amount of the catalyst used is generally 0.1 to 30 wt. % based on resorcin.

The reaction temperature is not particularly limited, but it is generally in a range of from 10° to 200°C. The reaction pressure also is not particularly limited, and generally, it is in a range of from atmospheric pressure to 150 atm.

For alkali, NaOH, KOH, LiOH, $Na_2CO_3$, etc. are used.

By acetylating 1,3-cyclohexanedione thus obtained, 2-acetyl-1,3-cyclohexanedione is obtained. For the acetylating agent, acetic anhydride or acetyl chloride is used.

By dehydrogenating 2-acetyl-1,3-cyclohexanedione by heating in a reaction medium in the presence of a palladium catalyst, 2-acetylresorcin is obtained. For the reaction medium used in this reaction, various kinds of solvent inert to the reaction are used.

Such solvent includes hydrocarbons (e.g. benzene, cumene, cymene, diisopropylbenzene, decalin, naphthalene, biphenyl), aliphatic ethers (e.g. diphenyl ether, diethylene glycol diethyl ether, triethylene glycol diethyl ether), aromatic ethers, etc. The solvent, however, is not limited to these examples, and also may be used in a mixture of two or more solvents.

The palladium may be present in an amount of from 0.0002 to 20%, say 0.2 to 20% by weight based on the weight of 2-acetyl-1,3-cyclohexanedione.

In carrying out the reaction of the present invention, it is advantageous to use a palladium catalyst as the catalyst. In such catalyst, palladium is generally used as supported on the various well-known carriers such as carbon (eg. graphite or activated carbon), silica, alumina, etc. Generally, the concentration of the palladium is preferably 0.1 to 10 wt. % based on the carrier.

The amount of such a palladium catalyst (including the carrier)used is generally 0.2 to 20 wt. % based on 2-acetyl-1,3-cyclohexanedione, a starting material.

The reaction temperature is not particularly limited, and generally, it is in a range of from 140° to 260°C. Temperatures below this range retard the reaction rate, and ones exceeding this range tend to lower the selectivity. The reaction pressure also is not particularly limited, and generally, it is in a range of from atmospheric pressure to 20 atm.

By this reaction, dehydrogenation of 2-acetyl-1,3-cyclohexanedione is attained, but the product is susceptible to hydrogenation by hydrogen produced as by-product during this reaction to produce 2-ethylresorcin, a by-product. In order to avoid this effect, it is desirable to pass an inert gas such as nitrogen gas or carbon dioxide gas through the reaction system to reduce the partial pressure of hydrogen.

This reaction has a tendancy to increase the selectivity to desired 2-acetylresorcin with a decrease in the conversion of the material.  It is also possible, therefore, to increase the yield by stopping the reaction at a stage wherein the conversion is low, recovering the desired product and re-using the unreacted material in cycle.  For example, this dehydrogenation can be repeated in such a manner that:  After dehydrogenation, the catalyst is filtered off, the filtrate is cooled, the precipitated crystals are separated to recover 2-acetylresorcin, and then the resulting mother liquor is re-used for dehydrogenation as a solvent containing the unreacted material.

2-Ethylresorcin, a by-product produced by this reaction, can selectively be removed into the aqueous layer, for example, by washing the mother liquor after separation of crystals with water.

After completion of the reaction, separation of 2-acetylresorcin from the reaction mixture is carried out, for example, by the common techniques wherein the reaction mixture is filtered to remove the catalyst, the filtrate is cooled as such or after removing a part of the solvent by distillation, and the precipitated crystals are separated.

Thus, according to the method of the present invention, 2-acetylresorcin can be produced industrially easily and in good yields.

The present invention will be illustrated with reference to the following examples, but it is not limited to these examples.

Example 1

To a 500-ml autoclave were added 110 g of resorcin, 250 g of a 25% aqueous sodium hydroxide solution and 4 g of paste-form Raney nickel, and after feeding hydrogen to a pressure of 40 kg/cm$^2$, the temperature was raised to 50°C with stirring. The hydrogen pressure lowered with the lapse of time, so that hydrogen was fed again to a pressure of 40 kg/cm$^2$ at the point when the pressure lowered below 10 kg/cm$^2$. This operation was repeated until a reduction in the hydrogen pressure was no longer observed, a reaction time of from 4 to 5 hours being required for this. After completion of the reaction, the contents of the autoclave were cooled to 30°C and Raney nickel was filtered off. To the filtrate were added 130 g of 35% aqueous hydrochloric acid, 126 g of sodium chloride and 200 g of water, and the mixture was cooled to 5°C. The precipitated crystals were filtered off and dried to obtain 103 g of 1,3-cyclohexanedione (yield, 92%).

Example 2

To a 500-ml flask equipped with a reflux condenser were added 103 g of 1,3-cyclohexanedione, 113 g of acetic anhydride and 14.8 g of sodium acetate, the contents of the flask were heated to 140°C, and reaction was carried out for 7 hours at the same temperature.

Thereafter, the contents of the flask were cooled to 40° to 50°C, diluted with 50 ml of water and extracted with 100 g of toluene. The toluene layer was washed with 50 ml of water and toluene was removed by distillation. The residue was rectified at 160°C under a pressure of 5 mmHg to obtain 92 g of 2-acetyl-1,3-cyclo-hexanedione (yield, 65%).

Examples 3 to 6

To a 300-ml flask equipped with a reflux condenser were added 5 g of 2-acetyl-1,3-cyclohexanedione, 1 g of 5% Pd-C catalyst and 100 g of each of the solvents described in Table 1, and the mixture was stirred at 175° to 200°C for 4 to 9 hours in a nitrogen gas atmosphere. After completion of the reaction, the catalyst was filtered off, and the filtrate was analyzed by gas chromatography to obtain the results shown in Table 1.

Table 1

| Example | Reaction condition | | | Conver-sion *2 | 2-Acetyl-resorcin | | 2-Ethyl-resorcin | |
|---|---|---|---|---|---|---|---|---|
| | Solvent | Reaction tempera-ture (°C) | Reaction time (hour) | | Yield *3 (%) | Selec-tivity *4 (%) | Yield *3 (%) | Selec-tivity *4 (%) |
| 3 | Diphenyl ether | 200 | 4 | 99 | 72 | 73 | 16 | 16 |
| 4 | Decalin *1 | 190 | 6 | 95 | 64 | 67 | 13 | 14 |
| 5 | p-Diiso-propyl-benzene | 200 | 5 | 98 | 66 | 67 | 15 | 15 |
| 6 | p-Cymene | 175 | 9 | 93 | 66 | 71 | 4.0 | 4.3 |

*1 Decalin : cis/trans = 64/36

$$\text{*2 Conversion (\%)} = \frac{\text{Fed 2-AcC (mole)} - \text{unreacted 2-AcC (mole)}}{\text{fed 2-AcC (mole)}} \times 100$$

$$= \frac{\text{Converted 2-AcC (mole)}}{\text{fed 2-AcC (mole)}} \times 100$$

$$\text{*3 Yield (\%)} = \frac{\text{Produced 2-acetylresorcin (or 2-ethylresorcin) (mole)}}{\text{fed 2-AcC (mole)}} \times 100$$

$$\text{*4 Selectivity (\%)} = \frac{\text{Produced 2-acetylresorcin (or 2-ethylresorcin) (mole)}}{\text{converted 2-AcC (mole)}} \times 100$$

wherein 2-AcC represents 2-acetyl-1,3-cyclohexanedione.

Example 7

Procedure was carried out in the same manner as in Example 6 except that 1.5 g of 5% Pd-$SiO_2$ was used as the catalyst. As a result, the followings were found: Conversion, 86%; yield of 2-acetylresorcin, 57%; selectivity of 2-acetylresorcin, 66%; yield of 2-ethylresorcin, 7%; and selectivity of 2-ethylresorcin, 8%.

Example 8

To the same flask as used in Example 1 were added 10 g of 2-acetyl-1,3-cyclohexanedione, 0.2 g of 5% Pd-C catalyst and 100 g of p-cymene, and the mixture was stirred at 175°C for 4 hours in a nitrogen gas atmosphere.

After completion of the reaction, the catalyst was filtered off, the filtrate was cooled, and the precipitated crystals were separated at 20°C. The resulting crude 2-acetylresorcin was washed with hexane and dried to obtain 4.36 g of 2-acetylresorcin (yield, 99%). The weight of the filtrate after separation of crystals was 105.2 g. It was found by gas-chromatographic analysis that this filtrate contained 0.4 g of 2-acetylresorcin, 0.1 g of 2-ethylresorcin and 4.8 g of the unreacted material. At the time of completion of the reaction, the followings were found: Conversion, 52%; yield of 2-acetylresorcin, 48%; selectivity of 2-acetylresorcin, 92%; yield of 2-ethylresorcin, 2%; and selectivity of 2-ethylresorcin, 4%.

0219282

Example 9

To 105.2 g of the filtrate after separation of crystals obtained in Example 8 were added 5.2 g of 2-acetyl-1,3-cyclohexanedione and 0.2 g of 5% Pd-C catalyst, and the mixture was stirred at 175°C for 4 hours in a nitrogen gas atmosphere in the same manner as in Example 8. After completion of the reaction, the catalyst was filtered off, and the filtrate was analyzed by gas chromatography to find that the filtrate contained 5.3 g of 2-acetylresorcin, 0.3 g of 2-ethylresorcin and 5.0 g of the unreacted material.

The results of this reaction described below show that the reaction of Example 8 was almost reproduced:  Conversion, 50%; yield of 2-acetylresorcin, 45%; selectivity of 2-acetylresorcin, 90%; yield of 2-ethylresorcin, 2%; and selectivity of 2-ethylresorcin, 4%.

WHAT IS CLAIMED IS:

1.      A method for producing 2-acetylresorcin which comprises dehydrogenating 2-acetyl-1,3-cyclohexanedione by heating in a reaction medium in the presence of a palladium catalyst.

2.      A method according to Claim 1, wherein the palladium catalyst is palladium supported on carbon.

3.      A method according to Claim 1 or 2, wherein the amount of the palladium catalyst used is 0.2 to 20 wt. % based on 2-acetyl-1,3-cyclohexanedione.

4.      A method according to any one of Claims 1 to 3, wherein the reaction temperature is in a range of from 140° to 260°C.

5.      A method according to any preceding Claim, wherein 2-acetyl-1,3-cyclohexanedione is produced by acetylating 1,3-cyclohexanedione.

6.      A method according to Claim 5, wherein the acetylating agent is acetic anhydride or acetyl chloride.

7.      A method according to Claim 5 or 6, wherein the 1,3-cyclohexanedione is produced by hydrogenating resorcin in an aqueous alkali solution in the presence of a catalyst.

8.      A method according to Claim 7, wherein the catalyst is a Raney nickel catalyst, ruthenium catalyst or rhodium catalyst.

9.      A method according to Claim 7 or 8, wherein the amount of the catalyst used is 0.1 to 30 wt. % based on resorcin.

10.     A method according to Claim 7, 8 or 9, wherein the alkali is a hydroxide or carbonate of an alkali metal.

11.      A method according to Claim 7, wherein the reaction temperature is in a range of from 100° to 200°C.

12.      A method according to any one of the preceding claims, wherein, during the dehydrogenation of the 2-acetyl-1,3-cyclohexanedione, an inert gas is passed through the reaction medium.